# EUROPEAN PATENT SPECIFICATION

(11) **EP 1 368 467 B1**
(45) Date of publication and mention of the grant of the patent: **25.04.2007**
(21) Application number: 01990934.0
(22) Date of filing: 07.11.2001
(51) Int. Cl.: C12N 15/82, C12N 5/14, A01H 5/00

(54) **PUTRESCINE-N-METHYLTRANSFERASE PROMOTER**
PUTRESCIN-N-METHYLTRANSFERASEPROMOTOR
PROMOTEUR DE LA PUTRESCINE-N-M THYLTRANSF RASE

(30) Priority: 07.11.2000 US 246488 P
(43) Date of publication of application: 10.12.2003
(60) Divisional application: 06122858.1
(73) Proprietor: NORTH CAROLINA STATE UNIVERSITY, Raleigh, North Carolina 27695-7003 (US)
(72) Inventor: CONKLING, Mark, A., Fuquay-Varina, NC 27707 (US); LI, Yan, Durham, NC 27713 (US)
(74) Representative: Harrison Goddard Foote
(86) International application number: PCT/US2001/047371
(87) International publication number: WO 2002/038588

(56) References cited:
- WO-A-00/67558
- SHOJI T ET AL: "Jasmonate induction of putrescine N-methyltransferase genes in the root of nicotiana sylvestris" PLANT AND CELL PHYSIOLOGY, JAPANESE SOCIETY OF PLANT PHYSIOLOGISTS, vol. 41, no. 7, 2000, pages 831-839, XP002974019 ISSN: 0032-0781
- HIBI N ET AL: "GENE EXPRESSION IN TOBACCO LOW-NICOTINE MUTANTS" PLANT CELL, AMERICAN SOCIETY OF PLANT PHYSIOLOGISTS, ROCKVILLE, MD, US, vol. 6, no. 5, May 1994 (1994-05), pages 723-735, XP001037067 ISSN: 1040-4651
- RIECHERS ET AL.: 'Structure and expression of the gene family encoding putrescine N-methyltransferase in Nicotiana tabacum: new clues to the evolutionary origin of cultivated tobacco' PLANT MOLECULAR BIOLOGY vol. 41, 1999, pages 387 - 401, XP002966768
- HASHIMOTO ET AL.: 'Intraspecific variability of the tanden repeats in Nicotiana putrescine N-methyltransferases' PLANT MOLECULAR BIOLOGY vol. 37, no. 1, 1998, pages 25 - 37, XP002966769

## Description

### Field of the Invention

The present invention concerns root-specific promoters useful in plants, along with methods of use thereof, constructs containing the same, and transgenic plants produced with such promoters.

### Background of the Invention

A promoter is generally defined as a nucleic acid sequence upsteam or downstream from a transcribed gene, and to which RNA polymerase must bind if it is to transcribe the flanking gene into messenger RNA. A promoter may consist of a number of different regulatory elements that affect a structural gene operationally associated with the promoter in different ways. For example, a regulatory element may enhance or repress expression of an associated structural gene, subject that gene to developmental regulation, or contribute to the tissue-specific regulation of that gene. Modifications to promoters can make possible optional patterns of gene expression, using recombinant DNA procedures. See, e.g., Old and Primrose, Principles of Gene Manipulation (4th Ed., 1989).

Transgenic plants expressing peptides that inhibit or kill a particular pest or pathogen provide a method for decreasing crop damage and loss. For example, expression of the *Bacillus thuringiensis* protein in transgenic corn provides resistance to the European corn borer. However, transgene expression in all tissues of a plant (constitutive expression) can be disadvantageous as it can expose non-target organisms to the transgenic protein and can increase the selective pressure for the development of pathogens and pests which are resistant to the transgenic protein. High levels of transgene expression throughout a plant may also negatively affect growth and yield of the plant. An alternative strategy is to express a toxic peptide only in the organ or tissue affected by a particular pest or pathogen. Implementation of this strategy against pests and pathogens that attack plant roots has been hampered by the lack of characterized root-specific promoters.

Transcription of a gene is initiated when a stable complex is formed between RNA polymerase enzyme and a gene promoter. Promoters generally occur at the beginning of all transcription units, are typically about 100 base pairs in length, and generally are located immediately upstream from the start site of transcription. See e.g., Maniatis et al., Science 236:1238 (1987). Promoters vary in their "strength"; that is, in their ability to accurately and efficiently initiate transcription. The RNA polymerase holoenzyme is thought to cover a region of about 50 bases immediately upstream of the transcribed region. In some cases the strength of transcription initiation may be enhanced by auxiliary proteins that bind adj acent to the region of the promoter which is immediately upstream from the transcribed DNA. See, e.g., Singer & Berg, Genes and Genomes, 140-145, University Science Books, Mill Valley, Cailf. (1991).

U.S. Patent No. 5,459,252 to Conkling and Yamamoto describes the RB7 root promoter.

U.S. Patent No. 5,837,876 to Conkling, Mendu and Song describes the RD2 root cortex specific promoter, also known as the NtQPT1 promoter.

### Summary of the Invention

A first aspect of the present invention is an isolated DNA molecule which directs root specific transcription of a downstream heterologous DNA segment in a plant cell. The promoter is a putrescine-N-methyltransferase (PMT) promoter, such as the tobacco PMT promoter or NtPMT1 promoter. The promoter of the present invention is an isolated DNA molecule having sequence SEQ ID NO: 1 provided herein, and which directs root specific transcription of a downstream heterologous DNA segment in a plant cell.

A further aspect of the present invention is an expression cassette comprising SEQ ID NO:1 and a heterologous DNA segment positioned downstream from, and operatively associated with, the promoter.

A further aspect of the present invention is an expression cassette comprising a root specific promote as defined in SEQ ID NO:1 and a heterologous DNA segment, the sequence of the root specific promoter as described herein.

Further aspects of the present invention are plant cells comprising the above described expression cassettes, methods of making transformed plants from such plant cells, and the transformed plants comprising such transformed plant cells.

### Brief Description of the Drawings

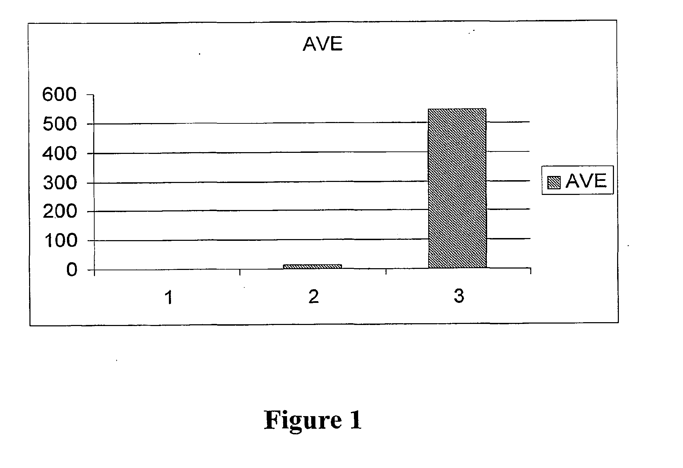

Figure 1 shows average GUS expression levels in leaves (column 1), stems (column 2), and roots (column 3) directed by the PMT promoter.

### Detailed Description of the Preferred Embodiments

Nucleotide sequences are presented herein by single strand only, in the 5' to 3' direction, from left to right Nucleotides are represented herein in the manner recommended by the IUPAC-IUB Biochemical Nomenclature Commission.

The root specific promoter of the present invention is a DNA molecule which has a sequence corresponding to SEQ ID NO: 1, which is discussed in greater detail below.

As used herein, a TobPMT promoter refers to a DNA molecule having a sequence identical to, or substantially homologous to, a continuous segment of the DNA found 5' to the transcribed region of the tobacco PMT gene. SEQ ID NO:1 given herein provides the sequence of the region found immediately 5' to the transcription start site in the TobPMT gene.

As used herein, a root specific promoter is a promoter that preferentially directs expression of an operatively associated DNA, nucleic acid or gene in root tissue, as compared to expression in leaf or stem tissue, or other tissues of the plant.

The root specific promoter of the present invention is useful in directing tissue specific expression of trans genes in transformed plants. Such tissue-specific transgene expression is useful in providing resistance against damage caused by pests and pathogens which attack plant roots. In addition, as the root is a major sink organ for photosynthate storage, expression of transgenes designed to alter the stored carbohydrates may be directed by such promoters. Exogenous genes of particular interest for root specific expression include those that code for proteins that bind heavy metals (such as metallothionein); proteins that give resistance to soil borne pests and pathogens; proteins that confer resistance to heat, salt (salinity) and drought; proteins for desalinization; and proteins that metabolize plant storage compounds into alternative preferred products or forms.

The root specific promoter of the invention may be used to express proteins or peptides in "molecular farming" applications. Such proteins or peptides include but are not limited to industrial enzymes, antibodies, therapeutic agents, and nutritional products. Such root-specific expression is particularly useful when the plant is a root crop plant such as a sugar beet.

The root specific promoter of the invention is also useful for expressing an oligonucleotide that will decrease or inhibit expression of a native gene in the plant. Such oligonucleotides may be from 4, 6 or 8 nucleotides to 40, 80 or 100 nucleotides in length, or more, and may encode antisense oligonucleotides, ribozymes, sense suppression agents, or other products that inhibit the expression of a native gene.

Tissue specific promoter may also be used to convert pro-pesticides to active forms in selected tissue sites. Hsu et al. Pestic. Sci., 44, 9 (1995) report the use of a chimeric gene comprising the root-specific promoter TobRB7 and the B-glucuronidase enzyme gene, to preferentially convert a pro-pesticide to an active form in roots. The inactive pro-pesticide (a glucuronide of hydroxymethyloxamyl) was applied to foliage and was then transported through plant phloem to roots, where it was converted to an active nematocidal form by glucuronidase.

Additionally, root-cortex specific promoters are useful for histological purposes, to identify or stain root-cortex tissue using a reporter gene such as B-glucurodinase.

The term "operatively associated," as used herein, refers to DNA sequences contained within a single DNA molecule which are associated so that the function of one is affected by the other. Thus, a promoter is operatively associated with a gene when it is capable of affecting the expression of that gene (i.e., the gene is under the transcriptional control of the promoter). The promoter is said to be "upstream" from the gene, which is in turn said to be "downstream" from the promoter.

DNA constructs, or "expression cassettes," of the present invention include, 5'-3' in the direction of transcription, the promoter of the present invention, a heterologous DNA segment operatively associated with the promoter, and, optionally, transcriptional and translational termination regions such as a termination signal and/or a polyadenylation region. These regulatory regions are preferably capable of operating in the transformed cells. The 3' termination region may be derived from the same gene as the transcriptional initiation region or from a different gene.

Plants may be divided into those lacking chlorophyll (such as fungi) and those containing chlorophyll (such as green algae, mosses); and further divided into those containing chlorophyll and having vascular tissue (such as ferns, gymnosperms, conifers, monocots and dicots). The latter group of plants includes those in which roots, stems and leaves may be present. As used herein, the term 'plant' encompasses all such organisms described above. As used herein, the term 'natural plant DNA' means DNA isolated from non-genetically altered, or untransformed, plants (for example, plant varieties which are produced by selective breeding).

As used herein, the term heterologous gene or heterologous DNA segment means a gene (or DNA segment) which is used to transform a cell by genetic engineering techniques, and which may not occur naturally in the cell. Structural genes are those portions of genes which comprise a DNA segment coding for a protein, polypeptide, or portion thereof, possibly including a ribosome binding site and/or a translational start codon, but lacking a promoter. The term can also refer to copies of a structural gene naturally found within a cell but artificially introduced. Structural genes may encode a protein not normally found in the plant cell in which the gene is introduced or in combination with the promoter to which it is operationally associated. Genes which may be operationally associated with a promoter of the present invention for expression in a plant species may be derived from a chromosomal gene, cDNA, a synthetic gene, or combinations thereof. As used herein, the term heterologous DNA segment also includes DNA segments coding for non-protein products, such as ribozymes or anti-sense RNAs. Antisense RNAs are well known (see, e.g., U.S. Pat. No. 4,801,540 (Calgene, Inc.)).

Genes of interest for use with the present invention in plants include those affecting a wide variety of phenotypic and non-phenotypic properties. Among the phenotypic properties are proteins, such as enzymes, which provide resistance to various environmental stresses, including but not limited to stress caused by dehydration (resulting from heat, salinity or drought), herbicides, toxic metals, trace elements, pests and pathogens. Resistance may be due to a change in the target site, enhancement of the amount of a target protein in the host cell, increased amounts of one or more enzymes involved with the biosynthetic pathway of a product which protects the host against the stress, and the like. Structural genes may be obtained from prokaryotes or eukaryotes, bacteria, fungi, (e.g., from yeast, viruses, plants, and mammals) or may be synthesized in whole or in part. Illustrative genes include glyphosphate resistant 3-enolpyruvylphosphoshikinate synthase gene, nitrilase, genes in the proline and glutamine biosynthetic pathway, and metallothioneins.

Structural genes operatively associated with the promoter of the present invention may be those which code for a protein toxic to insects, such as *a Bacillus thuringiensis* crystal protein toxic to insects. A DNA sequence encoding a *B*. *thuringiensis toxin* toxic to Coleoptera, and variations of this sequence wherein the toxicity is retained, is disclosed in U.S. Pat. No. 4,853,331 (see also U.S. Pat. Nos. 4,918,006 and 4,910,136)(the disclosures of all U.S. Patent references cited herein are to be incorporated herein in their entirety by reference). A gene sequence from *B*. *thuringiensis* which renders plant species toxic to Lepidoptera is disclosed in PCT Application WO 90/02804. PCT Application WO 89/04868 discloses transgenic plants transformed with a vector which promotes the expression of a *B. thuringiensis* crystal protein, the sequence of which may be employed in connection with the present invention. PCT Application WO 90/06999 discloses DNA encoding a *B*. *thuringiensis* crystal protein toxin active against Lepidoptera. Another gene sequence encoding an insecticidal crystal protein is disclosed in U.S. Pat. No. 4,918,006. Exemplary of gene sequences encoding other insect toxins are gene sequences encoding a chitinase (e.g., EC-3.2.1.14), as disclosed in U.S. Pat. No. 4,940,840 and PCT Appln. No. WO 90/07001. A gene coding for a nematode-inducible pore protein useful in producing transgenic plants resistant to root nematodes is disclosed in U.S. Pat. Application Ser. No. 08/007,998. Strains of *B. thuringiensis* which produce polypeptide toxins active against nematodes are disclosed in U.S. Pat. Nos. 4,948,734 and 5,093,120.

Where the expression product of the gene is to be located in a cellular compartment other than the cytoplasm, the structural gene may be constructed to include regions which code for particular amino acid sequences which result in translocation of the product to a particular site, such as the cell plasma membrane, or secretion into the periplasmic space or into the external environment of the cell. Various secretory leaders, membrane integration sequences, and translocation sequences for directing the peptide expression product to a particular site are described in the literature. See, for example, Cashmore et al., Biotechnology (1985) 3:803-808, Wickner and Lodish, Science (1985) 230:400-407.

The expression cassette may be provided in a DNA construct which also has at least one replication system. For convenience, it is common to have a replication system functional in *Escherichia coli,* such as ColE1, pSC101, pACYC184, or the like. In this manner, at each stage after each manipulation, the resulting construct may be cloned, sequenced, and the correctness of the manipulation determined. In addition, or in place of the *E. coli* replication system, a broad host range replication system may be employed, such as the replication systems of the P-1 incompatibility plasmids, e.g., pRK290. In addition to the replication system, there may be at least one marker present, which may be useful in one or more hosts, or different markers for individual hosts. That is, one marker may be employed for selection in a prokaryotic host while another marker may be employed for selection in a eukaryotic host, particularly the plant host. The markers may provide protection against a biocide, such as antibiotics, toxins, heavy metals, or the like; may provide complementation by imparting prototrophy to an auxotrophic host; or may provide a visible phenotype through the production of a novel compound in the plant. Exemplary genes which may be employed include neomycin phosphotransferase (NPTII), hygromycin phosphotransferase (HPT), chloramphenicol acetyltransferase (CAT), nitrilase, and the gentamicin resistance gene. For plant host selection, nonlimiting examples of suitable markers are beta-glucuronidase (GUS) (providing indigo production), luciferase (providing visible light production), NPTII (providing kanamycin resistance or G418 resistance), HPT (providing hygromycin resistance), and the mutated aroA gene (providing glyphosate resistance).

The various constructs, expression cassettes, markers, and the like may be introduced consecutively by restriction enzyme cleavage of an appropriate replication system and insertion of the particular construct or fragment into the available site. After ligation and cloning, the DNA construct may be isolated for further manipulation. All of these techniques are amply exemplified in the literature. See, e.g., Maniatis et al., Molecular Cloning: A Laboratory Manual, Cold Spring Harbor Laboratory, Cold Spring Harbor, N.Y. (1982).

A vector is a replicable DNA construct. Vectors which may be used to transform plant tissue with DNA constructs of the present invention include both Agrobacterium vectors and ballistic vectors, as well as vectors suitable for DNA-mediated transformation. Agrobacterium tumefaciens cells containing a DNA construct of the present invention, wherein the DNA construct comprises a Ti plasmid, are useful in methods of making transformed plants. Plant cells are infected with an Agrobacterium tumefaciens to produce a transformed plant cell, and then a plant is regenerated from the transformed plant cell.

Numerous Agrobacterium vector systems useful in carrying out the present invention are known. For example, U.S. Pat. No. 4,459,355 discloses a method for transforming susceptible plants, including dicots, with an Agrobacterium strain containing the Ti plasmid. The transformation of woody plants with an Agrobacterium vector is disclosed in U.S. Pat. No. 4,795,855. Further, U.S. Pat No. 4,940,838 to Schilperoort et al. discloses a binary Agrobacterium vector (i.e., one in which the Agrobacterium contains one plasmid having the vir region of a Ti plasmid but no T-DNA region, and a second plasmid having a T-DNA region but no vir region) useful in carrying out the present invention.

Microparticles carrying a DNA construct of the present invention, which microparticle is suitable for the ballistic transformation of a plant cell, are also useful for making transformed plants of the present invention. The microparticle is propelled into a plant cell to produce a transformed plant cell and a plant is regenerated from the transformed plant cell. Any suitable ballistic cell transformation methodology and apparatus can be used in practicing the present invention. Exemplary apparatus and procedures are disclosed in Sanford and Wolf, U.S. Pat. No. 4,945,050, and in Agracetus European Patent Application Publication No. 0 270 356, titled "Pollen-mediated Plant Transformation". When using, ballistic transformation procedures, the expression cassette may be incorporated into a plasmid capable of replicating in the cell to be transformed. Examples of microparticles suitable for use in such systems include 1 to 5 .mu.m gold spheres. The DNA construct may be deposited on the microparticle by any suitable technique, such as by precipitation.

A transformed host cell is a cell which has been transformed or transfected with constructs containing a DNA sequence as disclosed herein using recombinant DNA techniques. Plant species may be transformed with the DNA construct of the present invention by the DNA-mediated transformation of plant cell protoplasts and subsequent regeneration of the plant from the transformed protoplasts in accordance with procedures well known in the art.

The promoter sequence disclosed herein may be used to express a heterologous DNA sequence in any plant species capable of utilizing the promoter (i.e., any plant species the RNA polymerase of which binds to the promoter sequences disclosed herein). Examples of plant species suitable for transformation with the DNA constructs of the present invention include both monocots and dicots, and include but are not limited to tobacco, soybean, potato, cotton, sugarbeet, sunflower, carrot, celery, flax, cabbage and other cruciferous plants, pepper, tomato, citrus trees, bean, strawberry, lettuce, maize, alfalfa, oat, wheat, rice, barley, sorghum and canola. Thus an illustrative category of plants which may be transformed with the DNA construct of the present invention are the dicots, and a more particular category of plants which may be transformed using the DNA construct of the present invention are members of the family Solanacae.

Any plant tissue capable of subsequent clonal propagation, whether by organogenesis or embryogenesis, may be transformed with a vector of the present invention. The term "organogenesis," as used herein, means a process by which shoots and roots are developed sequentially from meristematic centers; the term "embryogenesis," as used herein, means a process by which shoots and roots develop together in a concerted fashion (not sequentially), whether from somatic cells or gametes. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristems, axillary buds, and root meristems), and induced meristem tissue (e.g., cotyledon meristem and hypocotyl meristem).

### EXAMPLE 1

### Cloning of the NtPMT Promoter

Using the *PMT* cDNA sequence published by N. Hibi et aL, *Plant Cell 6,* 723-725 (1994) we designed nested, divergent PCR primers for Inverse-PCR. Tobacco genomic DNA was cut with a variety of restriction endonucleases, ligated at low DNA concentrations (to promote circularization), and then used as a template for Inverse-PCR. The longest amplification product was obtained from genomic DNA that had been digested with *Nde*I*.* This fragment was cloned and sequenced. Sequence comparisons to the *PMT* cDNA showed sequence identity in the known regions, illustrating that the amplified DNA product was, indeed, the 5' flanking region of the *PMT* gene. The DNA sequence of the *NtPMT* promoter is as follows:

The sequence corresponding to the PMT cDNA published by Hibi et al. *supra,* is underlined. The initiating ATG is in bold. The sequence used as a primer for inverse-PCR is in italics.

### EXAMPLE 2

### Transgenic Plants

The *NtPMT1* promoter set forth in Example 1 (SEQ ID NO: 1) was fused to the *GUS* gene in pBI101 and transformed into tobacco in accordance with standard techniques. *See, e.g.,* U.S. Patent No. 5,837,876 at Examples 4-6. Transgenic tobacco was stained for GUS activity using X-Gluc. Transgenic roots stained for GUS activity.

### EXAMPLE 3

### Expression Analysis

**Figure 1** shows average GUS expression levels in leaves (column 1), stems (column 2), and roots (column 3) directed by the PMT promoter. Data for all 48 independent transformants we examined. There was substantial variation among the GUS levels from transformant to transformant. Important to note is the average GUS activity level (pmol MU/mg protein/min) for roots is 544.82, for stems is 13.24 (~40-fold enhancement in roots), and for leaves is 0.26 (~2000-fold enhancement in roots). Also note that the majority of leaves (44/48) and stems (40/48) had no GUS activity.

## Claims

1. An isolated DNA molecule which directs root specific transcription of a downstream heterologous DNA segment in a plant cell, said isolated DNA molecule having sequence SEQ ID NO:1.

2. A DNA construct comprising, in the 5' to 3' direction, a root specific promoter and a heterologous DNA segment downstream from said promoter and operatively associated therewith, wherein said root specific promoter has sequence SEQ ID NO:1.

3. A DNA construct according to claim 2, wherein said construct is a plasmid.

4. A DNA construct according to either claim 2 or 3, wherein said heterologous DNA segment is either a gene coding for an insecticidal protein or is a gene coding for a Bacillus thuringiensis crystal protein toxic to insects.

5. A plant cell transformed with, or a plant cell protoplast comprising, a DNA construct according any one of claims 2 to 4.

6. A method of making a transformed plant, comprising regenerating a plant from a plant cell or plant cell protoplast according to claim 5.

7. A cell containing a DNA construct according to any one of claims 2 to 4, and wherein said DNA construct further comprises a Ti plasmid.

8. A method of making a transformed plant, comprising infecting a plant cell with a construct according to claim 7 to produce a transformed plant cell, and then regenerating a plant from said transformed plant cell.

9. A microparticle carrying a DNA construct according to any one of claims 2 to 4, wherein said microparticle is suitable for the ballistic transformation of a plant cell.

10. A method of making a transformed plant, comprising propelling a microparticle according to claim 9 into a plant cell to produce a transformed plant cell, and then regenerating a plant from said transformed plant cell.

11. A transformed plant comprising transformed plant cells, said transformed plant cells comprising a heterologous DNA construct, which construct comprises in the 5' to 3' direction, a root specific promoter and a heterologous DNA segment downstream from said promoter and operatively associated therewith, said promoter having sequence SEQ ID NO.1

12. A transformed plant according to claim 11 wherein said plant is a dicot or a monocot or a tobacco plant.

13. A transformed plant according to claim 11 or claim 12 wherein the heterologous DNA segment encodes a protein that is toxic to insects.

## Patentansprüche

1. Isoliertes DNS-Molekül, welches eine Wurzel-spezifsche Transkription eines stromabwärts gelegenen heterologen DNS-Segments in einer Pflanzen-Zelle dirigiert, wobei das isolierte DNS-Molekül die Sequenz Seq.-ID Nr. 1 aufweist.

2. DNS-Konstrukt, umfassend - in der Richtung 5'nach 3' - einen Wurzel-spezifischen Promoter und ein heterologes DNS-Segment, das stromabwärts von dem Promoter liegt und damit operativ verbunden ist, worin der Wurzel-spezifische Promoter die Sequenz Seq.-ID Nr. 1 aufweist.

3. DNS-Konstrukt nach Anspruch 2, worin das Konstrukt ein Plasmid ist.

4. DNS-Konstrukt entweder nach Anspruch 2 oder nach Anspruch 3, worin das heterologe DNS-Segment entweder ein Gen ist, das für ein insektizides Protein kodiert, oder ein Gen ist, das für ein *Bacillus thuringiensis*-Kristall-Protein kodiert, das für Insekten toxisch ist.

5. Pflanzenzelle, transformiert mit einem, oder Pflanzenzelle-Protoplast, umfassend ein, DNS-Konstrukt nach einem der Ansprüche 2 bis 4.

6. Verfahren zur Herstellung einer transformierten Pflanze, umfassend ein Regenerieren einer Pflanze von einer Pflanzenzelle oder einem Pflanzenzellen-Protoplast nach Anspruch 5.

7. Zelle, enthaltend ein DNS-Konstrukt nach einem der Ansprüche 2 bis 4, und worin das DNS-Konstrukt weiter ein Ti-Plasmid umfasst.

8. Verfahren zur Herstellung einer transformierten Pflanze, umfassend ein Infizieren einer Pflanzenzelle mit einem Konstrukt nach Anspruch 7 unter Herstellung einer transformierten Pflanzenzelle und ein anschließendes Regenerieren einer Pflanze von der transformierten Pflanzenzelle.

9. Mikropartikel, tragend ein DNS-Konstrukt nach einem der Ansprüche 2 bis 4, worin der Mikropartikel für die ballistische Transformation einer Pflanzenzelle geeignet ist.

10. Verfahren zur Herstellung einer transformierten Pflanze, umfassend das Voranbringen eines Mikropartikels nach Anspruch 9 in eine Pflanzenzelle unter Herstellung einer transformierten Pflanzenzelle und ein anschließendes Regenerieren einer Pflanze von der transformierten Pflanzenzelle.

11. Transformierte Pflanze, umfassend transformierte Pflanzenzellen, wobei die transformierten Pflanzenzellen ein heterologes DNS-Konstrukt umfassen, wobei das Konstrukt in der Richtung 5' nach 3' einen Wurzel-spezifischen Promoter und ein heterologes DNS-Segment stromabwärts von dem Promoter und damit operativ verbunden umfasst, wobei der Promoter eine Sequenz Seq.-ID Nr. 1 aufweist.

12. Tranformierte Pflanze nach Anspruch 11, worin die Pflanzenzelle ein Dikotyle oder ein Monokotyle oder eine Tabakpflanze ist.

13. Transformierte Pflanze nach Anspruch 11 oder Anspruch 12, worin das heterologe DNS-Segment für ein Protein kodiert, das toxisch für Insekten ist.

## Revendications

1. Molécule d'ADN isolée qui dirige la transcription, spécifique des racines, d'un segment d'ADN hétérologue en aval dans une cellule végétale, ladite molécule d'ADN isolée ayant une séquence SEQ ID N° 1.

2. Produit d'assemblage d'ADN comprenant, dans le sens 5' à 3',
un promoteur spécifique des racines et un segment d'ADN hétérologue en aval du promoteur et associé de manière fonctionnelle à celui-ci, dans lequel ledit promoteur spécifique des racines a la séquence SEQ ID N° 1.

3. Produit d'assemblage d'ADN suivant la revendication 2, ledit produit d'assemblage étant un plasmide.

4. Produit d'assemblage d'ADN suivant la revendication 2 ou 3, dans lequel ledit segment d'ADN hétérologue est un gène codant pour une protéine insecticide ou bien est un gène codant pour une protéine cristalline de Bacillus thuringiensis toxique pour les insectes.

5. Cellule végétale transformée avec, ou protoplaste de cellule végétale comprenant, un produit d'assemblage d'ADN suivant l'une quelconque des revendications 2 à 4.

6. Procédé pour la production d'une plante transformée, comprenant la régénération d'une plante à partir d'une cellule végétale ou d'un protoplaste de cellule végétale suivant la revendication 5.

7. Cellule contenant un produit d'assemblage d'ADN suivant l'une quelconque des revendications 2 à 4, dans laquelle ledit produit d'assemblage d'ADN comprend en outre un plasmide Ti.

8. Procédé pour la production d'une plante transformée, comprenant l'infection d'une cellule végétale avec un produit d'assemblage suivant la revendication 7 pour produire une cellule végétale transformée, et ensuite la régénération d'une plante à partir de ladite cellule végétale transformée.

9. Microparticule portant un produit d'assemblage d'ADN suivant l'une quelconque des revendications 2 à 4, ladite microparticule convenant pour la transformation balistique d'une cellule végétale.

10. Procédé pour la production d'une plante transformée, comprenant la propulsion d'une microparticule suivant la revendication 9 dans une cellule végétale pour produire une cellule végétale transformée, et ensuite la régénération d'une plante à partir de ladite cellule végétale transformée.

11. Plante transformée comprenant des cellules végétales transformées, lesdites cellules végétales transformées comprenant un produit d'assemblage d'ADN hétérologue, produit d'assemblage qui comprend, dans le sens 5' à 3', un promoteur spécifique des racines et un segment d'ADN hétérologue en aval dudit promoteur et associé de manière fonctionnelle à celui-ci, ledit promoteur ayant la séquence SEQ ID N° 1.

12. Plante transformée suivant là revendication 11, ladite plante étant une plante dicotylédone ou monocotylédone ou un plant de tabac.

13. Plante transformée suivant la revendication 11 ou la revendication 12, dans laquelle le segment d'ADN hétérologue code pour une protéine qui est toxique pour les insectes.


### SEQUENCE LISTING

<110> Conkling, Mark
   Li, Yan
<120> Putrescine-N-Methyltransferase Promoter
<130> 5051-530
<140> To be assigned
   <141> 2001-11-06
<150> US 60/246,488
   <151> 2000-11-07
<160> 11
<170> PatentIn version 3.1
<210> 1
   <211> 742
   <212> DNA
   <213> NtPMT promoter
<400> 1
   gtataccaaa aatcaattca acccccaaaa cataatacaa ccaatgttaa tgcaatatct    60
   ctgctgctat cacgaagata attgtagctc acgaaagtag gatacattat gtaggttaca   120
   tcacatagag gtaatctaaa gctcccaata ataagatgtg taatgttgat tatgtagaaa   180
   tttgccaggt tatttagaat aaacaagaag aggagaaaaa aagtacaatt tacctgaact   240
   cttgaatgta tcctacaaat aacctagact tcatggacgt cagttgtcag tttacttttg   300
   ttttaatggt acatcatttg tcaaatactt tatttggata aaaacagttt tgcctaagga   360
   gtaaacagat ccggagtaag aaagcagacg attaaagcaa tttttaaaaa aggagagaga   420
   aattaatgag cacacacata tactagtgaa attagggtac taatttacta ataattgcac   480
   cgagacaaac ttatatttta gttccaaaat gtcagtctaa ccctgcacgt tgtaataaat   540
   ttttaactct attatattat atcgagttgc gccctccact cctcggtgtc caaattgtat   600
   ttaaatgcat agatgtttaa tgggagtgta cagcaagctt tcggaaaata caaaccataa   660
   tactttctct tcttcaattt gtttagttta attttgaaaa tggaagtcat atctaccaac   720
   acaaatggct ctaccatctt ca                                            742
<210> 2
   <211> 732
   <212> DNA
   <213> Deletion mutant of PMT promoter
<400> 2
   aatcaattca acccccaaaa cataatacaa ccaatgttaa tgcaatatct ctgctgctat 60
   cacgaagata attgtagctc acgaaagtag gatacattat gtaggttaca tcacatagag 120
   gtaatctaaa gctcccaata ataagatgtg taatgttgat tatgtagaaa tttgccaggt 180
   tatttagaat aaacaagaag aggagaaaaa aagtacaatt tacctgaact cttgaatgta 240
   tcctacaaat aacctagact tcatggacgt cagttgtcag tttacttttg ttttaatggt 300
   acatcatttg tcaaatactt tatttggata aaaacagttt tgcctaagga gtaaacagat 360
   ccggagtaag aaagcagacg attaaagcaa tttttaaaaa aggagagaga aattaatgag 420
   cacacacata tactagtgaa attagggtac taatttacta ataattgcac cgagacaaac 480
   ttatatttta gttccaaaat gtcagtctaa ccctgcacgt tgtaataaat ttttaactct 540
   attatattat atcgagttgc gccctccact cctcggtgtc caaattgtat ttaaatgcat 600
   agatgtttaa tgggagtgta cagcaagctt tcggaaaata caaaccataa tactttctct 660
   tcttcaattt gtttagttta attttgaaaa tggaagtcat atctaccaac acaaatggct 720
   ctaccatctt ca 732
<210> 3
   <211> 722
   <212> DNA
   <213> Deletion mutant of PMT promoter
<400> 3
   acccccaaaa cataatacaa ccaatgttaa tgcaatatct ctgctgctat cacgaagata 60
   attgtagctc acgaaagtag gatacattat gtaggttaca tcacatagag gtaatctaaa 120
   gctcccaata ataagatgtg taatgttgat tatgtagaaa tttgccaggt tatttagaat 180
   aaacaagaag aggagaaaaa aagtacaatt tacctgaact cttgaatgta tcctacaaat 240
   aacctagact tcatggacgt cagttgtcag tttacttttg ttttaatggt acatcatttg 300
   tcaaatactt tatttggata aaaacagttt tgcctaagga gtaaacagat ccggagtaag 360
   aaagcagacg attaaagcaa tttttaaaaa aggagagaga aattaatgag cacacacata 420
   tactagtgaa attagggtac taatttacta ataattgcac cgagacaaac ttatatttta 480
   gttccaaaat gtcagtctaa ccctgcacgt tgtaataaat ttttaactct attatattat 540
   atcgagttgc gccctccact cctcggtgtc caaattgtat ttaaatgcat agatgtttaa 600
   tgggagtgta cagcaagctt tcggaaaata caaaccataa tactttctct tcttcaattt 660
   gtttagttta attttgaaaa tggaagtcat atctaccaac acaaatggct ctaccatctt 720
   ca 722
<210> 4
   <211> 712
   <212> DNA
   <213> Deletion mutant of PMT promoter
<400> 4
   cataatacaa ccaatgttaa tgcaatatct ctgctgctat cacgaagata attgtagctc     60
   acgaaagtag gatacattat gtaggttaca tcacatagag gtaatctaaa gctcccaata    120
   ataagatgtg taatgttgat tatgtagaaa tttgccaggt tatttagaat aaacaagaag    180
   aggagaaaaa aagtacaatt tacctgaact cttgaatgta tcctacaaat aacctagact    240
   tcatggacgt cagttgtcag tttacttttg ttttaatggt acatcatttg tcaaatactt    300
   tatttggata aaaacagttt tgcctaagga gtaaacagat ccggagtaag aaagcagacg    360
   attaaagcaa tttttaaaaa aggagagaga aattaatgag cacacacata tactagtgaa    420
   attagggtac taatttacta ataattgcac cgagacaaac ttatatttta gttccaaaat    480
   gtcagtctaa ccctgcacgt tgtaataaat ttttaactct attatattat atcgagttgc    540
   gccctccact cctcggtgtc caaattgtat ttaaatgcat agatgtttaa tgggagtgta    600
   cagcaagctt tcggaaaata caaaccataa tactttctct tcttcaattt gtttagttta    660
   attttgaaaa tggaagtcat atctaccaac acaaatggct ctaccatctt ca            712
<210> 5
   <211> 702
   <212> DNA
   <213> Deletion mutant of PMT promoter
<400> 5
   ccaatgttaa tgcaatatct ctgctgctat cacgaagata attgtagctc acgaaagtag     60
   gatacattat gtaggttaca tcacatagag gtaatctaaa gctcccaata ataagatgtg    120
   taatgttgat tatgtagaaa tttgccaggt tatttagaat aaacaagaag aggagaaaaa    180
   aagtacaatt tacctgaact cttgaatgta tcctacaaat aacctagact tcatggacgt    240
   cagttgtcag tttacttttg ttttaatggt acatcatttg tcaaatactt tatttggata    300
   aaaacagttt tgcctaagga gtaaacagat ccggagtaag aaagcagacg attaaagcaa    360
   tttttaaaaa aggagagaga aattaatgag cacacacata tactagtgaa attagggtac    420
   taatttacta ataattgcac cgagacaaac ttatatttta gttccaaaat gtcagtctaa    480
   ccctgcacgt tgtaataaat ttttaactct attatattat atcgagttgc gccctccact    540
   cctcggtgtc caaattgtat ttaaatgcat agatgtttaa tgggagtgta cagcaagctt    600
   tcggaaaata caaaccataa tactttctct tcttcaattt gtttagttta attttgaaaa    660
   tggaagtcat atctaccaac acaaatggct ctaccatctt ca                       702
<210> 6
   <211> 692
   <212> DNA
   <213> Deletion mutant of PMT promoter
<400> 6
   tgcaatatct ctgctgctat cacgaagata attgtagctc acgaaagtag gatacattat     60
   gtaggttaca tcacatagag gtaatctaaa gctcccaata ataagatgtg taatgttgat    120
   tatgtagaaa tttgccaggt tatttagaat aaacaagaag aggagaaaaa aagtacaatt    180
   tacctgaact cttgaatgta tcctacaaat aacctagact tcatggacgt cagttgtcag    240
   tttacttttg ttttaatggt acatcatttg tcaaatactt tatttggata aaaacagttt    300
   tgcctaagga gtaaacagat ccggagtaag aaagcagacg attaaagcaa tttttaaaaa    360
   aggagagaga aattaatgag cacacacata tactagtgaa attagggtac taatttacta    420
   ataattgcac cgagacaaac ttatatttta gttccaaaat gtcagtctaa ccctgcacgt    480
   tgtaataaat ttttaactct attatattat atcgagttgc gccctccact cctcggtgtc    540
   caaattgtat ttaaatgcat agatgtttaa tgggagtgta cagcaagctt tcggaaaata    600
   caaaccataa tactttctct tcttcaattt gtttagttta attttgaaaa tggaagtcat    660
   atctaccaac acaaatggct ctaccatctt ca                                  692
<210> 7
   <211> 642
   <212> DNA
   <213> Deletion mutant of PMT promoter
<400> 7
   gtataccaaa aatcaattca acccccaaaa cataatacaa ccaatgttaa tgcaatatct     60
   ctgctgctat cacgaagata attgtagctc acgaaagtag gatacattat gtaggttaca    120
   tcacatagag gtaatctaaa gctcccaata ataagatgtg taatgttgat tatgtagaaa    180
   tttgccaggt tatttagaat aaacaagaag aggagaaaaa aagtacaatt tacctgaact    240
   cttgaatgta tcctacaaat aacctagact tcatggacgt cagttgtcag tttacttttg    300
   ttttaatggt acatcatttg tcaaatactt tatttggata aaaacagttt tgcctaagga    360
   gtaaacagat ccggagtaag aaagcagacg attaaagcaa tttttaaaaa aggagagaga    420
   aattaatgag cacacacata tactagtgaa attagggtac taatttacta ataattgcac    480
   cgagacaaac ttatatttta gttccaaaat gtcagtctaa ccctgcacgt tgtaataaat    540
   ttttaactct attatattat atcgagttgc gccctccact cctcggtgtc caaattgtat    600
   ttaaatgcat agatgtttaa tgggagtgta cagcaagctt tc                       642
<210> 8
   <211> 630
   <212> DNA
   <213> Deletion mutant of PMT promoter
<400> 8
   gtataccaaa aatcaattca acccccaaaa cataatacaa ccaatgttaa tgcaatatct 60
   ctgctgctat cacgaagata attgtagctc acgaaagtag gatacattat gtaggttaca 120
   tcacatagag gtaatctaaa gctcccaata ataagatgtg taatgttgat tatgtagaaa 180
   tttgccaggt tatttagaat aaacaagaag aggagaaaaa aagtacaatt tacctgaact 240
   cttgaatgta tcctacaaat aacctagact tcatggacgt cagttgtcag tttacttttg 300
   ttttaatggt acatcatttg tcaaatactt tatttggata aaaacagttt tgcctaagga 360
   gtaaacagat ccggagtaag aaagcagacg attaaagcaa tttttaaaaa aggagagaga 420
   aattaatgag cacacacata tactagtgaa attagggtac taatttacta ataattgcac 480
   cgagacaaac ttatatttta gttccaaaat gtcagtctaa ccctgcacgt tgtaataaat 540
   ttttaactct attatattat atcgagttgc gccctccact cctcggtgtc caaattgtat 600
   ttaaatgcat agatgtttaa tgggagtgta 630
<210> 9
   <211> 620
   <212> DNA
   <213> Deletion mutant of PMT promoter
<400> 9
   gtataccaaa aatcaattca acccccaaaa cataatacaa ccaatgttaa tgcaatatct 60
   ctgctgctat cacgaagata attgtagctc acgaaagtag gatacattat gtaggttaca 120
   tcacatagag gtaatctaaa gctcccaata ataagatgtg taatgttgat tatgtagaaa 180
   tttgccaggt tatttagaat aaacaagaag aggagaaaaa aagtacaatt tacctgaact 240
   cttgaatgta tcctacaaat aacctagact tcatggacgt cagttgtcag tttacttttg 300
   ttttaatggt acatcatttg tcaaatactt tatttggata aaaacagttt tgcctaagga 360
   gtaaacagat ccggagtaag aaagcagacg attaaagcaa tttttaaaaa aggagagaga 420
   aattaatgag cacacacata tactagtgaa attagggtac taatttacta ataattgcac 480
   cgagacaaac ttatatttta gttccaaaat gtcagtctaa ccctgcacgt tgtaataaat 540
   ttttaactct attatattat atcgagttgc gccctccact cctcggtgtc caaattgtat 600
   ttaaatgcat agatgtttaa 620
<210> 10
   <211> 630
   <212> DNA
   <213> Deletion mutant of PMT promoter
<400> 10
   aatcaattca acccccaaaa cataatacaa ccaatgttaa tgcaatatct ctgctgctat     60
   cacgaagata attgtagctc acgaaagtag gatacattat gtaggttaca tcacatagag    120
   gtaatctaaa gctcccaata ataagatgtg taatgttgat tatgtagaaa tttgccaggt    180
   tatttagaat aaacaagaag aggagaaaaa aagtacaatt tacctgaact cttgaatgta    240
   tcctacaaat aacctagact tcatggacgt cagttgtcag tttacttttg ttttaatggt    300
   acatcatttg tcaaatactt tatttggata aaaacagttt tgcctaagga gtaaacagat    360
   ccggagtaag aaagcagacg attaaagcaa tttttaaaaa aggagagaga aattaatgag    420
   cacacacata tactagtgaa attagggtac taatttacta ataattgcac cgagacaaac    480
   ttatatttta gttccaaaat gtcagtctaa ccctgcacgt tgtaataaat ttttaactct    540
   attatattat atcgagttgc gccctccact cctcggtgtc caaattgtat ttaaatgcat    600
   agatgtttaa tgggagtgta cagcaagctt                                      630
<210> 11
   <211> 610
   <212> DNA
   <213> Deletion mutant of PMT promoter
<400> 11
   acccccaaaa cataatacaa ccaatgttaa tgcaatatct ctgctgctat cacgaagata     60
   attgtagctc acgaaagtag gatacattat gtaggttaca tcacatagag gtaatctaaa    120
   gctcccaata ataagatgtg taatgttgat tatgtagaaa tttgccaggt tatttagaat    180
   aaacaagaag aggagaaaaa aagtacaatt tacctgaact cttgaatgta tcctacaaat    240
   aacctagact tcatggacgt cagttgtcag tttacttttg ttttaatggt acatcatttg    300
   tcaaatactt tatttggata aaaacagttt tgcctaagga gtaaacagat ccggagtaag    360
   aaagcagacg attaaagcaa tttttaaaaa aggagagaga aattaatgag cacacacata    420
   tactagtgaa attagggtac taatttacta ataattgcac cgagacaaac ttatatttta    480
   gttccaaaat gtcagtctaa ccctgcacgt tgtaataaat ttttaactct attatattat    540
   atcgagttgc gccctccact cctcggtgtc caaattgtat ttaaatgcat agatgtttaa    600
   tgggagtgta                                                            610

### SEQUENCE LISTING

<110> Conkling, Mark A.
   Li, Yan
<120> Putrescine N-Methyltransferase Promoter
<130> 5051-530WO
<140> PCT/US 01/47371
   <141> 2001-11-07
<150> US 60/246488
   <151> 2000-11-07
<160> 11
<170> PatentIn version 3.1
<210> 1
   <211> 742
   <212> DNA
   <213> NtPMT promoter
<400> 1
   gtataccaaa aatcaattca acccccaaaa cataatacaa ccaatgttaa tgcaatatct 60
   ctgctgctat cacgaagata attgtagctc acgaaagtag gatacattat gtaggttaca 120
   tcacatagag gtaatctaaa gctcccaata ataagatgtg taatgttgat tatgtagaaa 180
   tttgccaggt tatttagaat aaacaagaag aggagaaaaa aagtacaatt tacctgaact 240
   cttgaatgta tcctacaaat aacctagact tcatggacgt cagttgtcag tttacttttg 300
   ttttaatggt acatcatttg tcaaatactt tatttggata aaaacagttt tgcctaagga 360
   gtaaacagat ccggagtaag aaagcagacg attaaagcaa tttttaaaaa aggagagaga 420
   aattaatgag cacacacata tactagtgaa attagggtac taatttacta ataattgcac 480
   cgagacaaac ttatatttta gttccaaaat gtcagtctaa ccctgcacgt tgtaataaat 540
   ttttaactct attatattat atcgagttgc gccctccact cctcggtgtc caaattgtat 600
   ttaaatgcat agatgtttaa tgggagtgta cagcaagctt tcggaaaata caaaccataa 660
   tactttctct tcttcaattt gtttagttta attttgaaaa tggaagtcat atctaccaac 720
   acaaatggct ctaccatctt ca 742
<210> 2
   <211> 732
   <212> DNA
   <213> Deletion mutant of PMT promoter
<400> 2
   aatcaattca acccccaaaa cataatacaa ccaatgttaa tgcaatatct ctgctgctat      60
   cacgaagata attgtagctc acgaaagtag gatacattat gtaggttaca tcacatagag     120
   gtaatctaaa gctcccaata ataagatgtg taatgttgat tatgtagaaa tttgccaggt     180
   tatttagaat aaacaagaag aggagaaaaa aagtacaatt tacctgaact cttgaatgta     240
   tcctacaaat aacctagact tcatggacgt cagttgtcag tttacttttg ttttaatggt     300
   acatcatttg tcaaatactt tatttggata aaaacagttt tgcctaagga gtaaacagat     360
   ccggagtaag aaagcagacg attaaagcaa tttttaaaaa aggagagaga aattaatgag     420
   cacacacata tactagtgaa attagggtac taatttacta ataattgcac cgagacaaac     480
   ttatatttta gttccaaaat gtcagtctaa ccctgcacgt tgtaataaat ttttaactct     540
   attatattat atcgagttgc gccctccact cctcggtgtc caaattgtat ttaaatgcat     600
   agatgtttaa tgggagtgta cagcaagctt tcggaaaata caaaccataa tactttctct     660
   tcttcaattt gtttagttta attttgaaaa tggaagtcat atctaccaac acaaatggct     720
   ctaccatctt ca                                                         732
<210> 3
   <211> 722
   <212> DNA
   <213> Deletion mutant of PMT promoter
<400> 3
   acccccaaaa cataatacaa ccaatgttaa tgcaatatct ctgctgctat cacgaagata 60
   attgtagctc acgaaagtag gatacattat gtaggttaca tcacatagag gtaatctaaa 120
   gctcccaata ataagatgtg taatgttgat tatgtagaaa tttgccaggt tatttagaat 180
   aaacaagaag aggagaaaaa aagtacaatt tacctgaact cttgaatgta tcctacaaat 240
   aacctagact tcatggacgt cagttgtcag tttacttttg ttttaatggt acatcatttg 300
   tcaaatactt tatttggata aaaacagttt tgcctaagga gtaaacagat ccggagtaag 360
   aaagcagacg attaaagcaa tttttaaaaa aggagagaga aattaatgag cacacacata 420
   tactagtgaa attagggtac taatttacta ataattgcac cgagacaaac ttatatttta 480
   gttccaaaat gtcagtctaa ccctgcacgt tgtaataaat ttttaactct attatattat 540
   atcgagttgc gccctccact cctcggtgtc caaattgtat ttaaatgcat agatgtttaa 600
   tgggagtgta cagcaagctt tcggaaaata caaaccataa tactttctct tcttcaattt 660
   gtttagttta attttgaaaa tggaagtcat atctaccaac acaaatggct ctaccatctt 720
   ca 722
<210> 4
   <211> 712
   <212> DNA
<213> Deletion mutant of PMT promoter
<400> 4
   cataatacaa ccaatgttaa tgcaatatct ctgctgctat cacgaagata attgtagctc 60
   acgaaagtag gatacattat gtaggttaca tcacatagag gtaatctaaa gctcccaata 120
   ataagatgtg taatgttgat tatgtagaaa tttgccaggt tatttagaat aaacaagaag 180
   aggagaaaaa aagtacaatt tacctgaact cttgaatgta tcctacaaat aacctagact 240
   tcatggacgt cagttgtcag tttacttttg ttttaatggt acatcatttg tcaaatactt 300
   tatttggata aaaacagttt tgcctaagga gtaaacagat ccggagtaag aaagcagacg 360
   attaaagcaa tttttaaaaa aggagagaga aattaatgag cacacacata tactagtgaa 420
   attagggtac taatttacta ataattgcac cgagacaaac ttatatttta gttccaaaat 480
   gtcagtctaa ccctgcacgt tgtaataaat ttttaactct attatattat atcgagttgc 540
   gccctccact cctcggtgtc caaattgtat ttaaatgcat agatgtttaa tgggagtgta 600
   cagcaagctt tcggaaaata caaaccataa tactttctct tcttcaattt gtttagttta 660
   attttgaaaa tggaagtcat atctaccaac acaaatggct ctaccatctt ca 712
<210> 5
   <211> 702
   <212> DNA
   <213> Deletion mutant of PMT promoter
<400> 5
   ccaatgttaa tgcaatatct ctgctgctat cacgaagata attgtagctc acgaaagtag     60
   gatacattat gtaggttaca tcacatagag gtaatctaaa gctcccaata ataagatgtg    120
   taatgttgat tatgtagaaa tttgccaggt tatttagaat aaacaagaag aggagaaaaa    180
   aagtacaatt tacctgaact cttgaatgta tcctacaaat aacctagact tcatggacgt    240
   cagttgtcag tttacttttg ttttaatggt acatcatttg tcaaatactt tatttggata    300
   aaaacagttt tgcctaagga gtaaacagat ccggagtaag aaagcagacg attaaagcaa    360
   tttttaaaaa aggagagaga aattaatgag cacacacata tactagtgaa attagggtac    420
   taatttacta ataattgcac cgagacaaac ttatatttta gttccaaaat gtcagtctaa    480
   ccctgcacgt tgtaataaat ttttaactct attatattat atcgagttgc gccctccact    540
   cctcggtgtc caaattgtat ttaaatgcat agatgtttaa tgggagtgta cagcaagctt    600
   tcggaaaata caaaccataa tactttctct tcttcaattt gtttagttta attttgaaaa    660
   tggaagtcat atctaccaac acaaatggct ctaccatctt ca                        702
<210> 6
   <211> 692
<212> DNA
   <213> Deletion mutant of PMT promoter
<400> 6
   tgcaatatct ctgctgctat cacgaagata attgtagctc acgaaagtag gatacattat      60
   gtaggttaca tcacatagag gtaatctaaa gctcccaata ataagatgtg taatgttgat     120
   tatgtagaaa tttgccaggt tatttagaat aaacaagaag aggagaaaaa aagtacaatt     180
   tacctgaact cttgaatgta tcctacaaat aacctagact tcatggacgt cagttgtcag     240
   tttacttttg ttttaatggt acatcatttg tcaaatactt tatttggata aaaacagttt     300
   tgcctaagga gtaaacagat ccggagtaag aaagcagacg attaaagcaa tttttaaaaa     360
   aggagagaga aattaatgag cacacacata tactagtgaa attagggtac taatttacta     420
   ataattgcac cgagacaaac ttatatttta gttccaaaat gtcagtctaa ccctgcacgt     480
   tgtaataaat ttttaactct attatattat atcgagttgc gccctccact cctcggtgtc     540
   caaattgtat ttaaatgcat agatgtttaa tgggagtgta cagcaagctt tcggaaaata     600
   caaaccataa tactttctct tcttcaattt gtttagttta attttgaaaa tggaagtcat     660
   atctaccaac acaaatggct ctaccatctt ca                                   692
<210> 7
   <211> 642
   <212> DNA
   <213> Deletion mutant of PMT promoter
<400> 7
   gtataccaaa aatcaattca acccccaaaa cataatacaa ccaatgttaa tgcaatatct      60
   ctgctgctat cacgaagata attgtagctc acgaaagtag gatacattat gtaggttaca     120
   tcacatagag gtaatctaaa gctcccaata ataagatgtg taatgttgat tatgtagaaa     180
   tttgccaggt tatttagaat aaacaagaag aggagaaaaa aagtacaatt tacctgaact     240
   cttgaatgta tcctacaaat aacctagact tcatggacgt cagttgtcag tttacttttg     300
   ttttaatggt acatcatttg tcaaatactt tatttggata aaaacagttt tgcctaagga     360
   gtaaacagat ccggagtaag aaagcagacg attaaagcaa tttttaaaaa aggagagaga     420
   aattaatgag cacacacata tactagtgaa attagggtac taatttacta ataattgcac     480
   cgagacaaac ttatatttta gttccaaaat gtcagtctaa ccctgcacgt tgtaataaat     540
   ttttaactct attatattat atcgagttgc gccctccact cctcggtgtc caaattgtat     600
   ttaaatgcat agatgtttaa tgggagtgta cagcaagctt tc                        642
<210> 8
   <211> 630
   <212> DNA
<213> Deletion mutant of PMT promoter
<400> 8
   gtataccaaa aatcaattca acccccaaaa cataatacaa ccaatgttaa tgcaatatct     60
   ctgctgctat cacgaagata attgtagctc acgaaagtag gatacattat gtaggttaca    120
   tcacatagag gtaatctaaa gctcccaata ataagatgtg taatgttgat tatgtagaaa    180
   tttgccaggt tatttagaat aaacaagaag aggagaaaaa aagtacaatt tacctgaact    240
   cttgaatgta tcctacaaat aacctagact tcatggacgt cagttgtcag tttacttttg    300
   ttttaatggt acatcatttg tcaaatactt tatttggata aaaacagttt tgcctaagga    360
   gtaaacagat ccggagtaag aaagcagacg attaaagcaa tttttaaaaa aggagagaga    420
   aattaatgag cacacacata tactagtgaa attagggtac taatttacta ataattgcac    480
   cgagacaaac ttatatttta gttccaaaat gtcagtctaa ccctgcacgt tgtaataaat    540
   ttttaactct attatattat atcgagttgc gccctccact cctcggtgtc caaattgtat    600
   ttaaatgcat agatgtttaa tgggagtgta                                     630
<210> 9
   <211> 620
   <212> DNA
   <213> Deletion mutant of PMT promoter
<400> 9
   gtataccaaa aatcaattca acccccaaaa cataatacaa ccaatgttaa tgcaatatct     60
   ctgctgctat cacgaagata attgtagctc acgaaagtag gatacattat gtaggttaca    120
   tcacatagag gtaatctaaa gctcccaata ataagatgtg taatgttgat tatgtagaaa    180
   tttgccaggt tatttagaat aaacaagaag aggagaaaaa aagtacaatt tacctgaact    240
   cttgaatgta tcctacaaat aacctagact tcatggacgt cagttgtcag tttacttttg    300
   ttttaatggt acatcatttg tcaaatactt tatttggata aaaacagttt tgcctaagga    360
   gtaaacagat ccggagtaag aaagcagacg attaaagcaa tttttaaaaa aggagagaga    420
   aattaatgag cacacacata tactagtgaa attagggtac taatttacta ataattgcac    480
   cgagacaaac ttatatttta gttccaaaat gtcagtctaa ccctgcacgt tgtaataaat    540
   ttttaactct attatattat atcgagttgc gccctccact cctcggtgtc caaattgtat    600
   ttaaatgcat agatgtttaa                                                620
<210> 10
   <211> 630
   <212> DNA
   <213> Deletion mutant of PMT promoter
<400> 10
   aatcaattca acccccaaaa cataatacaa ccaatgttaa tgcaatatct ctgctgctat      60
   cacgaagata attgtagctc acgaaagtag gatacattat gtaggttaca tcacatagag     120
   gtaatctaaa gctcccaata ataagatgtg taatgttgat tatgtagaaa tttgccaggt     180
   tatttagaat aaacaagaag aggagaaaaa aagtacaatt tacctgaact cttgaatgta     240
   tcctacaaat aacctagact tcatggacgt cagttgtcag tttacttttg ttttaatggt     300
   acatcatttg tcaaatactt tatttggata aaaacagttt tgcctaagga gtaaacagat     360
   ccggagtaag aaagcagacg attaaagcaa tttttaaaaa aggagagaga aattaatgag     420
   cacacacata tactagtgaa attagggtac taatttacta ataattgcac cgagacaaac     480
   ttatatttta gttccaaaat gtcagtctaa ccctgcacgt tgtaataaat ttttaactct     540
   attatattat atcgagttgc gccctccact cctcggtgtc caaattgtat ttaaatgcat     600
   agatgtttaa tgggagtgta cagcaagctt                                      630
<210> 11
   <211> 610
   <212> DNA
   <213> Deletion mutant of PMT promoter
<400> 11
   acccccaaaa cataatacaa ccaatgttaa tgcaatatct ctgctgctat cacgaagata     60
   attgtagctc acgaaagtag gatacattat gtaggttaca tcacatagag gtaatctaaa    120
   gctcccaata ataagatgtg taatgttgat tatgtagaaa tttgccaggt tatttagaat    180
   aaacaagaag aggagaaaaa aagtacaatt tacctgaact cttgaatgta tcctacaaat    240
   aacctagact tcatggacgt cagttgtcag tttacttttg ttttaatggt acatcatttg    300
   tcaaatactt tatttggata aaaacagttt tgcctaagga gtaaacagat ccggagtaag    360
   aaagcagacg attaaagcaa tttttaaaaa aggagagaga aattaatgag cacacacata    420
   tactagtgaa attagggtac taatttacta ataattgcac cgagacaaac ttatatttta    480
   gttccaaaat gtcagtctaa ccctgcacgt tgtaataaat ttttaactct attatattat    540
   atcgagttgc gccctccact cctcggtgtc caaattgtat ttaaatgcat agatgtttaa    600
   tgggagtgta                                                           610